# EUROPÄISCHE PATENTSCHRIFT

(11) **EP 1 150 681 B1**
(45) Veröffentlichungstag und Bekanntmachung des Hinweises auf die Patenterteilung: **04.01.2006**
(21) Anmeldenummer: 00905035.2
(22) Anmeldetag: 08.02.2000
(51) Int. Cl.: A61K 31/519, A61P 25/34

(54) **VERWENDUNG EINER PHARMAZEUTISCHE ZUSAMMENSETZUNG ENTHALTEND DESOXYPEGANIN ZUR BEHANDLUNG DER NIKOTINABHÄNGIGKEIT**
USE OF A PHARMACEUTICAL COMPOSITION CONTAINING DESOXYPEGANINE FOR THE TREATMENT OF NICOTINE DEPENDENCE
UTILISATION D' UNE COMPOSITION PHARMACEUTIQUE CONTENANT DE LA DESOXYPEGANINE UTILISEE POUR LE TRAITEMENT DE LA DEPENDANCE A LA NICOTINE

(30) Priorität: 19.02.1999 DE 19906979
(43) Veröffentlichungstag der Anmeldung: 07.11.2001
(73) Patentinhaber: HF ARZNEIMITTELFORSCHUNG GmbH, 59368 Werne (DE)
(72) Erfinder: ASMUSSEN, Bodo, D-56170 Bendorf (DE); HILLE, Thomas, D-56567 Neuwied (DE); HOFFMANN, Hans-Rainer, D-56566 Neuwied (DE); OPITZ, Klaus, D-48157 Münster (DE)
(74) Vertreter: Flaccus, Rolf-Dieter
(86) Internationale Anmeldenummer: PCT/EP2000/000975
(87) Internationale Veröffentlichungsnummer: WO 2000/048445

(56) Entgegenhaltungen:
- WO-A-00/48579
- DE-A- 19 906 974
- DE-A- 19 906 975
- TULYAGANOV, N. ET AL: "The pharmacological characteristics of deoxypeganine hydrochloride" FARMAKOL. TOKSIKOL. (MOSCOW) (1986), 49(3), 37-40 , XP000957689
- MURATOVA, V. V. ET AL: "Toxicology of the new pharmaceutical preparation dehydroxypeganine hydrochloride" MED. ZH. UZB. (1984), (1), 53-5 , XP000957819
- DATABASE WPI Section Ch, Week 197913 Derwent Publications Ltd., London, GB; Class B02, AN 1979-25213B XP002151986 & SU 605 614 A (AS UZB CHEM GROWING), 6. April 1978 (1978-04-06) in der Anmeldung erwähnt

## Beschreibung

Die vorliegende Erfindung bezieht sich auf die neue Verwendung eines wenig bekannten Wirkstoffs zur Behandlung der Nikotinabhängigkeit.

Insbesondere ist die vorliegende Erfindung auf die pharmazeutische Verwendung in Formulierungen gerichtet, durch die Desoxypeganin oder eines seiner pharmazeutisch annehmbaren Säureadditionssalze zur Behandlung der Nikotinabhängigkeit auf kontrollierte, z. B. kontinuierliche Weise abgegeben werden.

Die Abhängigkeit von Nikotin erfüllt alle von der WHO definierten Kriterien der Drogenabhängigkeit:
- zwanghafter Gebrauch
- psychoaktive Effekte
- Einfluss auf das Verhalten
- stereotype Konsumgewohnheiten
- Abstinenzerscheinungen bei Entzug oder Toleranzentwicklungen.

Rauchen ist also keine "schlechte Angewohnheit" und kann nicht in allen Fällen durch Willensstärke allein unterdrückt werden. Pharmakologen haben im Gehirn Nikotin-Rezeptoren gefunden, die biologische Erklärung dafür sind, dass so viele Raucher trotz starker Motivation und guter psychologischer Unterstützung immer wieder rückfällig werden.

Diese Erkenntnis führte 1975 zu einem völlig neuen Therapieansatz, der Nikotinzufuhr durch Kaugummi. Zunächst begeistert begrüsst, zeigte das System jedoch recht bald Schwachstellen. So wurden der bittere Geschmack und die geringe soziale Akzeptanz des Kaugummis bemängelt. Auch ist es bei diesen Systemen durchaus zu Missbrauch durch Überdosierung gekommen.

Alle diese Nachteile führten zur Entwicklung von transdermalen Systemen, die Nikotin enthalten, wie sie z. B. in der deutschen Patentschrift DE 36 29 304 und dem US-Patent 4,597,961 beschrieben sind.

Bei der transdermalen Gabe von Nikotin spielt Geschmack keine Rolle, die Anwendung ist unsichtbar, die Abgabe des Wirkstoffs erfolgt ohne orale Ersatzbefriedigung und Plasmaspitzen werden vermieden.

Als Nebenwirkung werden Hautirritationen an den Applikationsstellen beobachtet, Rötungen, leichte Schwellungen und Juckreiz, die in einigen Fällen zur Aufgabe der Therapie führten.

Nachteilig bei dieser Nikotintherapie ist darüber hinaus, dass bei dieser Art der Behandlung der extremen Giftigkeit des Nikotins keine Rechnung getragen wird.

Es besteht daher eine Bedarf an Medikamenten, die die Symptome der Nikotinabhängigkeit sicher unterdrücken, ohne dass aber die therapeutischen Dosen des Wirkstoffs eine Toxizität besitzen, die der des Nikotins vergleichbar ist.

Bisher wurden zur Behandlung der Nikotinabhängigkeit Stoffe aus folgenden Gruppen eingesetzt:
- Naturstoffe ohne Stickstoff, z. B. γ-Pyrone, Citronensäure, Essigsäure, Campher, Glucose, Vitamine, Terpene u. a.
- Alkaloide mit unterschiedlichem Wirkungsspektrum, z. B. Lobelin, Coffein, Galanthamin und Apocynaceen-Alkaloide
- tricyclische Antidepressiva wie z. B. Fluoxetin
- Clonidin
- Pyrrolopyrimidin

Schon aus der Verschiedenartigkeit der Therapieprinzipien erkennt man, dass ein wirksames Medikament zur Behandlung der Nikotinabhängigkeit noch nicht gefunden wurde, das nicht so toxisch ist wie Nikotin.

Aufgabe der Erfindung ist daher die Bereitstellung eines Arzneistoffs in einer oralen, transdermalen oder anderweitig parenteralen Formulierung, die diesen Arzneistoff möglichst kontrolliert freisetzt und gewährleistet, dass das Verlangen nach Nikotin gemindert wird. Der Begriff parenteral soll also alle Anwendungsformen, ausser der oralen, umfassen wie die rektale, intravenöse, intramuskuläre, intraperitoneale und nasale Anwendung.

Diese Aufgabe wird erfindungsgemäss in überraschender Weise gelöst durch die Verwendung von Desoxypeganin und / oder eines seiner pharmazeutisch annehmbaren Säureadditionssalze zur Behandlung der Nikotinabhängigkeit.

Diese Lösung ist umso erstaunlicher, als Desoxypeganin in der ehemaligen Sowjetunion zwar ausführlich untersucht und seine pharmakologischen Wirkungen intensiv erforscht worden sind, die erfindungsgemässe Verwendung einer desoxypeganin-haltigen Formulierung zur Behandlung der Nikotinabhängigkeit aber bisher nicht beschrieben wurde.

Aufgrund seiner pharmakologischen Eigenschaften gehört Desoxypeganin zur Gruppe der reversibel wirkenden Cholinesterasehemmstoffe, steht in seinen Wirkungen dem Physostigmin und dem Neostigmin nahe, zeichnet sich jedoch durch besondere spezifische Eigenschaften aus. Desoxypeganin hemmt nämlich nicht nur die Acetylcholinesterase, sondern auch die Monoaminoxydase.

Dieser Vorteil wiegt seine auf die Gewichtseinheit bezogene etwas geringere Cholinesterasehemmwirkung (im Vergleich zu Physostigmin) auf.

Im Gegensatz zu Neostigmin überwindet Desoxypeganin die Bluthirnschranke und antagonisiert die cerebralen Wirkungen cholinerger Gifte.

Die Gewinnung des Desoxypeganin erfolgt durch Isolierung aus der Steppenraute (Peganum harmala) oder durch Synthese.

Arzneiformen, die Wirkstoffe kontrolliert freisetzen, sind im Stand der Technik bereits bekannt. Die Verabreichung pharmazeutisch wirksamer Verbindungen mittels solcher Formulierungen kann oral, transdermal oder anderweitig parenteral erfolgen. In derartigen Arzneimitteln kann das Desoxypeganin als solches oder in Form pharmazeutisch annehmbarer Säureadditonssalze vorliegen z. B. als Hydrohalogenid, insbesondere - chlorid oder -bromid, oder als Salz einer anderen pharmazeutisch annehmbaren Säure. Diese Mittel enthalten femer in der Regel Hilfsstoffe, wie Trägerstoffe, Fliessverbesserer, Lösungsmittel und Öle, deren Art und Menge je nach Darbietungsform schwankt. Im allgemeinen liegt der Gehalt an Wirkstoff im Arzneimittel, berechnet als freies Desoxypeganin, zwischen 0,1 und 50 Gew.-%, vorzugsweise zwischen 2 und 15 Gew.-%.

Einige im Rahmen der vorliegenden Erfindung geeignete Formulierungen zur oralen Verabreichung sollen kurz beschrieben werden.

In einer solchen Formulierung ist der pharmazeutische Wirkstoff z. B. in einer semipermeablen Membran eingekapselt, wie z. B. in Zelluloseacetat. Mit einem Bohrer oder Laser wird in das Kapselmaterial ein winziges Loch gebohrt. Im Körper des Patienten, der behandelt wird, wird durch das Kapselmaterial Wasser absorbiert. Der pharmazeutische Wirkstoff wird durch osmotischen Druck in der gewünschten allmählichen, konstanten und kontrollierten Weise durch die kleine Öffnung getrieben. Solche Systeme sind beispielsweise in den US-Patenten US 3,760,805 und US 3,987,790 beschrieben. In diesen Systemen können die pharmazeutischen Wirkstoffe in fester Form oder absorbiert an Ionenaustauscher-Harze vorliegen.

Ein anderes System zur oralen Verabreichung wird von Sheth und Leeson im US-Patent 4 137 300 beschrieben. Dieses Patent beschreibt eine Formulierung, die eine Wachsmatrix enthält.

Die Wirkstoffe der vorliegenden Erfindung werden mittels entsprechender Formulierungen auf passende und geeignete Weise verabreicht. Die festen Wirkstoffe können in Lösung oder als Suspension verabreicht werden. Das Lösungs- oder Suspensionsmedium kann wässrig oder organisch sein. Geeignete Lösungs- oder Suspensionsmedien für Desoxypeganin sind z. B. Wasser, Silikonfluid oder Mineralöl.

Um die Verabreichung einer Verbindung mittels einer Formulierung wie vorstehend beschrieben zu vereinfachen, kann dem System ein Fliessverbesserer zugesetzt werden. Einige geeignete Fliessverbesserer für orale Formulierungen umfassen beispielsweise Polyethylenglykol, Hydroxypropylmethylcellulose und Zucker.

In einer Formulierung zur transdermalen Verabreichung von Verbindungen gemäss der vorliegenden Erfindung kann der pharmazeutische Wirkstoff in einer Matrix enthalten sein, von der er in der gewünschten allmählichen, konstanten und kontrollierten Weise abgegeben wird. Die Durchlässigkeit der Matrix bei der Freisetzung der Verbindung beruht auf Diffusion. Ein derartiges System ist in dem deutschen Patent 33 15 272 (US 4 769 028) beschrieben. Dieses System besteht aus einer undurchlässigen Rückschicht, einem damit verbundenen, besonders aufgebauten übersättigten WirkstoffReservoir aus einer Polymermatrix, einer mit dem Reservoir verbundenen, für den Wirkstoff durchlässigen Haftklebeschicht und einer die Haftklebeschicht abdeckenden, zum Gebrauch wieder ablösbaren Schutzschicht. Auch Systeme, in denen die Reservoirschicht eine so hohe Eigenklebrigkeit aufweist, dass sie gleichzeitig die Haftklebeschicht darstellt, sind möglich.

Das deutsche Patent DE 38 43 239 (US 5 089 267) beschreibt ein solches System.

Wenn der Wirkstoff durch die Haut absorbiert wird, erhält der zu Behandelnde auf diese Weise einen kontrollierten und vorbestimmbaren Zufluss des Wirkstoffes.

Andere geeignete transdermale Formulierungen sind in den US-Patenten 3,742,951, 3,797,494, 3,996,934 und 4,031,894 beschrieben. Diese Formulierungen bestehen grundsätzlich aus einer Rückschicht, die eine der Oberflächen darstellt, einer für den Wirkstoff durchlässigen Klebschicht, die die andere Oberfläche darstellt und letztlich einem Reservoir, das den Wirkstoff zwischen den beiden die Oberfläche bildenden Schichten enthält. Alternativ dazu kann der Wirkstoff auch in ein Vielzahl von Mikrokapseln enthalten sein, die in der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut oder Schleimhaut des zu Behandelnden steht, kontinuierlich abgegeben. Im Falle von Mikrokapseln kann das Kapselmaterial auch als Membran wirken.

Formulierungen, die zur anderweitigen parenteralen Applikation von Desoxypeganin und seinen Salzen in Frage kommen, sind solche, die eine Depotwirkung des Wirkstoffs ermöglichen. Hierbei wird die Formulierung als Injektionslösung auf nichtwässriger Grundlage appliziert. Die möglichen Lösungsmittel sind dem Fachmann bekannt. Als Beispiele seien die vegetabilischen Öle erwähnt, die einzelne Pharmakopöen vorschreiben, wie Erdnussöl, Olivenöl, Mandelöl, Sonnenblumenöl, Sojabohnenöl und Sesamöl. Rizinusöl zeigt oftmals eine besonders günstige Löslichkeit für Arzneimittel; daneben sind auch Öle tierischen Ursprungs geeignet.

Die Öle sind physiologisch indifferent und gut verträglich. Voraussetzung hierfür ist, dass sie besonders gereinigt sind und niedrige Säure- und Peroxidzahlen aufweisen. Da eine intravenöse Applikation wegen der fehlenden Mischbarkeit mit dem Blutserum nicht möglich ist und zu Lungenembolie führen kann, ist ihre Anwendung lediglich für intramuskuläre und subkutane Injektionspräparate möglich. Ölige Lösungen und Suspensionen verbleiben recht lange am Ort der Applikation (oft bis zu 1 Monat) und geben die Wirkstoffe protrahiert frei.

Die Dosierung des Desoxypeganins oder seiner pharmazeutisch annehmbaren Säureadditionssalze muss so hoch sein und über eine so lange Zeit erfolgen, dass eine nachhaltige Wirkung erzielt wird, und bedarf einer individuellen Einstellung.

Die Erfindung wird durch das folgende Beispiel erläutert:

### Beispiel: Einfluss von Desoxypeganin auf das Rauchen von gesunden Probanden

Im Rahmen der Erprobung einer desoxypeganinhaltigen Formulierung, die in vivo ca. 50 mg Desoxypeganin-Hydrochlorid pro Tag abgibt, wurden neben anderen Probanden auch zwei Raucher als Versuchspersonen herangezogen, da "Rauchen" kein Ausschlusskriterium bei der Prüfung darstellte. Überraschenderweise trat bei den beiden Rauchern das Phänomen auf, dass das Verlangen nach Zigaretten auffällig unterdrückt wurde. Die Anwendungsdauer betrug 24 Stunden. Die Daten sind in der folgenden Tabelle aufgeführt:

**Tabelle 1: Einfluß von Desoxypeganin-HCl auf Raucher**

| | Zigarettenkonsum ohne Desoxypeganin | Zigarettenkonsum nach Gabe von 50 mg Desoxypeganin-HCl/Tag |
|---|---|---|
| männlicher Proband | 22 Zigaretten (x von n=3)/Tag | 3 Zigaretten |
| weiblicher Proband | 41 Zigaretten (x von n=5)/Tag | 6 Zigaretten |

| | | |
|---|---|---|
| (x = Mittelwert) | | |

Wie die Tabelle zeigt, bewirkte schon die einmalige Gabe von 50 mg Desoxypeganin /Tag eine beträchtliche Reduktion des Zigarettenkonsums.

Man kann also festhalten, dass Desoxypeganin und seine pharmazeutisch annehmbaren Säureadditionssalze zur Behandlung der Nikotinabhängigkeit verwendet werden können. Diese Substanzen werden vorzugsweise in einer kontinuierlichen und kontrollierten Weise verabreicht. Die pharmazeutische Darreichungsform ermöglicht eine kontrollierte Freisetzung für z. B. orale, transdermale oder auf anderem Wege parenterale Verabreichung.

## Patentansprüche

1. Verwendung von Desoxypeganin und / oder einem seiner pharmazeutisch annehmbaren Säureadditionssalze zur Herstellung eines Arzneimittels zur Behandlung der Nikotinabhängigkeit.

2. Verwendung nach Anspruch 1, **dadurch gekennzeichnet, dass** Desoxypeganin in Anteilen von 0,1 bis 50 Gew.-%, vorzugsweise 2 bis 15 Gew.-%, berechnet als freies Desoxypeganin, in pharmazeutischen Zusammensetzungen enthalten ist.

3. Verwendung nach Anspruch 1 oder 2, **dadurch gekennzeichnet, dass** der Wirkstoff in einer oral applizierbaren Form vorliegt.

4. Verwendung nach Anspruch 1 oder 2, **dadurch gekennzeichnet, dass** der Wirkstoff in einer parenteral applizierbaren Form vorliegt.

5. Verwendung nach Anspruch 1 oder 2, **dadurch gekennzeichnet, dass** der Wirkstoff in einer transdermal applizierbaren Form vorliegt.

## Claims

1. The use of deoxypeganine and/or one of its pharmaceutically acceptable acid addition salts for the production of a medicament for treating nicotine dependence.

2. The use as claimed in claim 1, wherein deoxypeganine is contained in pharmaceutical compositions in proportions of 0.1 to 50% by weight, preferably 2 to 15% by weight, calculated as free deoxypeganine.

3. The use as claimed in claim 1 or 2, wherein the active compound is present in an orally administrable form.

4. The use as claimed in claim 1 or 2, wherein the active compound is present in a parenterally administrable form.

5. The use as claimed in claim 1 or 2, wherein the active compound is present in a transdermally administrable form.

## Revendications

1. Utilisation de désoxypéganine et/ou d'un de ses sels pharmaceutiquement acceptables pour la préparation d'un médicament destiné au traitement de la dépendance à la nicotine.

2. Utilisation selon la revendication 1, **caractérisée en ce que** la désoxypéganine est contenue dans des compositions pharmaceutiques en des proportions de 0,1 à 50% en poids, de préférence de 2 à 15% en poids, calculées comme désoxypéganine libre.

3. Utilisation selon la revendication 1 ou 2, **caractérisée en ce que** la substance active se trouve sous une forme pouvant être administrée par voie orale.

4. Utilisation selon la revendication 1 ou 2, **caractérisée en ce que** la substance active se trouve sous une forme pouvant être administrée par voie parentérale.

5. Utilisation selon la revendication 1 ou 2, **caractérisée en ce que** la substance active se trouve sous une forme pouvant être administrée par voie transdermique.
